# EUROPEAN PATENT SPECIFICATION

(11) **EP 3 803 690 B1**
(45) Date of publication and mention of the grant of the patent: **09.08.2023**
(21) Application number: 19815985.7
(22) Date of filing: 27.05.2019
(51) Int. Cl.: G06V 40/13, A61B 5/00

(54) **ACOUSTIC BIOMETRIC IMAGING SYSTEM WITH ACOUSTIC IMPEDANCE MATCHED OPAQUE MASKING LAYER, AND MANUFACTURING METHOD**
SYSTEM ZUR AKUSTISCHEN BIOMETRISCHEN BILDGEBUNG MIT AN AKUSTISCHER IMPEDANZ ANGEPASSTER LICHTUNDURCHLÄSSIGER MASKENSCHICHT UND HERSTELLUNGSVERFAHREN
SYSTÈME D'IMAGERIE BIOMÉTRIQUE ACOUSTIQUE À COUCHE DE MASQUAGE OPAQUE ADAPTÉE À L'IMPÉDANCE ACOUSTIQUE, ET PROCÉDÉ DE FABRICATION

(30) Priority: 04.06.2018 SE 1850677
(43) Date of publication of application: 14.04.2021
(73) Proprietor: Fingerprint Cards Anacatum IP AB, 411 19 Göteborg (SE)
(72) Inventor: LUNDAHL, Karl, 413 21 GÖTEBORG (SE); NILSSON, Hanna, 412 49 GÖTEBORG (SE); BENSON, Adam, 442 48 KUNGÄLV (SE); GRIP, Martin, 236 38 HÖLLVIKEN (SE)
(74) Representative: Kransell & Wennborg KB
(86) International application number: PCT/SE2019/050485
(87) International publication number: WO 2019/235988

(56) References cited:
- WO-A1-2013/056239
- WO-A1-2017/192895
- WO-A1-2018/047710
- WO-A2-2015/127335
- US-A1- 2011 234 545
- US-A1- 2011 234 545
- US-A1- 2013 234 994
- MEIRE, H. B et al.: "Ultrasound", Basic Ultrasound, 1995, pages 10-17, Chichester, UK ISBN: 0 471 916919

## Description

### Field of the Invention

The present invention relates to an acoustic biometric imaging system.

### Background of the Invention

Biometric systems are widely used as means for increasing the convenience and security of personal electronic devices, such as mobile phones etc. Fingerprint sensing systems, in particular, are now included in a large proportion of all newly released personal communication devices, such as mobile phones.

Due to their excellent performance and relatively low cost, capacitive fingerprint sensors are used in an overwhelming majority of all biometric systems.

Among other fingerprint sensing technologies, ultrasonic sensing also has the potential to provide advantageous performance, such as the ability to acquire fingerprint (or palmprint) images from very moist fingers etc.

One class of ultrasonic fingerprint systems of particular interest are systems in which acoustic signals are transmitted along a surface of a device member to be touched by a user, and a fingerprint (palmprint) representation is determined based on received acoustic signals resulting from the interaction between the transmitted acoustic signals and an interface between the device member and the user's skin.

Such ultrasonic fingerprint sensing systems, which are, for example, generally described in US 2017/0053151 may provide for controllable resolution, and allow for a larger sensing area, which may be optically transparent, without the cost of the fingerprint sensing system necessarily scaling with the sensing area.

Although the general principle of such ultrasonic fingerprint sensing is known, there appear to be remaining challenges to be overcome. For instance, it would be desirable to improve sensing of a finger placed on a transparent portion of a device, using at least one ultrasonic transducer that is not visible to the user of the device.

WO2017/192895 discloses a fingerprint sensing system including an array of ultrasonic transducers for sensing the fingerprint.

US 2011/234545 relates to an acoustic touch apparatus.

### Summary

In view of above-mentioned and other drawbacks of the prior art, it is an object of the present invention to provide for improved sensing of a finger placed on a transparent portion of a device, using at least one ultrasonic transducer that is not visible to the user of the device.

According to a first aspect of the present invention, it is therefore provided an acoustic biometric imaging system defined by claim 1.

It should be noted that the finger touch region is spaced apart from the first transducer region, so that the first ultrasonic transducer, which is arranged on the second side of the transparent device member, is not directly opposite the finger touch region on the first side of the transparent device member.

When the acoustic biometric imaging system according to embodiments of the present invention is in use, an acoustic transmit signal is transmitted by a transmitting ultrasonic transducer acoustically coupled to the transparent device member. The acoustic transmit signal is laterally propagated by the transparent device member, and interacts with a hand surface touching the transparent device member to produce acoustic interaction signals indicative of interactions between the acoustic transmit signal and an interface between the first face of the transparent device member and the hand surface touching the first face of the transparent device member. The interaction signals are laterally propagated by the transparent device member, and received by a receiving ultrasonic transducer acoustically coupled to the transparent device member. Based on the interaction signals, a representation of the contact area between the transparent device member and the hand surface can be determined. The representation of the contact area (such as a fingerprint) may be used to identify or authenticate the user using, per se, known methods.

The transmitting ultrasonic transducer and the receiving ultrasonic transducer may be different transducers. Alternatively, the same ultrasonic transducer may first transmit the acoustic transmit signal, and then receive acoustic interaction signals.

Other acoustic biometric imaging systems exist, in which the finger touch region is directly opposite the transducer region, so that the acoustic transmit signal is propagated directly through the transparent device member from the second side of the transparent device member to the first side of the transparent device member. Such systems have the obvious disadvantage that the finger touch region is predefined and relatively small, since it has to correspond to a region populated with ultrasonic transducers.

The present inventors have found that the lateral propagation of the acoustic transmit signal and the acoustic interaction signals in the transparent device member requires a significantly more efficient/better acoustic coupling between the ultrasonic transducer and the transparent device member than in existing acoustic biometric imaging systems of the above-described kind, in which the finger touch region is directly opposite the transducer region.

In particular, the present inventors have found that a conventional opaque masking layer formed by polymer ink cannot provide sufficient acoustic coupling between the ultrasonic transducer(s) and a transparent device member of materials typically used in modern electronic devices, to allow lateral propagation, by the transparent device member, of transmit signal and interactions signals as described further above.

The present invention is thus based upon the realization that this type of acoustic biometric image system requires the opaque masking layer used for hiding the ultrasonic transducer(s) to exhibit an acoustic impedance that is between the acoustic impedance of the ultrasonic transducer(s) and the acoustic impedance of the transparent device member.

According to various embodiments, the third acoustic impedance of the opaque masking layer may advantageously be greater than 8 MRayls and less than 24 MRayls.

To provide for a sufficiently high degree of acoustic energy transfer into the transparent device member, the opaque masking layer may advantageously have a thickness of less than around 10 µm.

To achieve the desired acoustic impedance, while still being opaque, the opaque masking layer may advantageously be a vacuum deposited layer. In other words, the opaque masking layer may have been formed by a vacuum deposition method, rather than through, for example, screen printing. It should be noted that it will be straight-forward for one of ordinary skill in the art to determine if a layer has been formed by a vacuum deposition method or, for example, has been screen printed.

The present inventors have found that, using suitable vacuum depositing techniques, the acoustic impedance of the opaque masking layer can be tuned to a desired value, while at the same time achieving one of several different colors. This is beneficial, especially for applications where esthetics is important, such as mobile personal devices etc.

An example of a suitable vacuum deposition technique for forming the opaque masking layer has been found to be so-called Non-Conductive Vacuum Metallization (NCVM), which is, per se, well known in the art for other applications.

According to various embodiments, furthermore, the opaque masking layer may advantageously be an oxide layer.

In embodiments, such an oxide layer may advantageously comprise silicon and zirconium. In particular, the present inventors have found that an opaque masking layer with an acoustic impedance tuned to work well with advantageous combinations of transducer materials (ceramics, such as PZT) and transparent device member materials (chemically strengthened glass, such as so-called gorilla glass) can be achieved by varying the ratio of silicon oxide to zirconium oxide in in the opaque masking layer. If a higher acoustic impedance is desired, the proportion of zirconium oxide should be higher, and vice versa.

According to the invention, the acoustic biometric imaging system comprises an attachment layer between the opaque masking layer and the first ultrasonic transducer.

The attachment layer may advantageously have an acoustic impedance that is also between the acoustic impedance of the transparent device member and the acoustic impedance of the first ultrasonic transducer. The attachment layer is made of a Bismuth-based alloy, such as a Sn-Bi alloy.

According to various embodiments, the acoustic biometric imaging system may advantageously further comprise a metallic layer between the opaque masking layer and the attachment layer.

The metallic layer may comprise a metal that can form a mechanically robust alloy together with the attachment layer. In the case when the attachment layer is made of SnBi, the metallic layer may thus, for example, be made of Ni or Cu.

According to embodiments, moreover, the first ultrasonic transducer may be a ceramic piezo-electric transducer. For instance, the first ultrasonic transducer may be made of PZT.

In embodiments, the first ultrasonic transducer may be configured to be a shear wave transducer. For example, a ceramic piezo-electric transducer may be appropriately poled.

In other embodiments, the first ultrasonic transducer may be configured to be a longitudinal wave transducer.

According to various embodiments, the acoustic biometric imaging system may further comprise transducer control circuitry connected to the first ultrasonic transducer for receiving, from the first ultrasonic transducer, electrical signals indicative of the acoustic signals conducted by the transparent device member from the finger touch region.

The transducer control circuitry may further be controllable to provide electrical signals to the first ultrasonic transducer (and/or to a second ultrasonic transducer) to cause the first ultrasonic transducer (and/or second ultrasonic transducer) to transmit the above-mentioned acoustic transmit signal.

The acoustic biometric imaging system may further comprise processing circuitry connected to the transducer control circuitry and configured to form a representation of the finger surface based on signals from the transducer control circuitry.

Moreover, the acoustic biometric imaging system according to various embodiments of the present invention may advantageously be included in an electronic device, further comprising a controller configured to: acquire the representation of the finger surface from the acoustic biometric imaging system; authenticate a user based on the representation; and perform at least one user-requested process only if the user is authenticated based on the representation.

### Brief Description of the Drawings

These and other aspects of the present invention will now be described in more detail, with reference to the appended drawings showing an example embodiment of the invention, wherein:
Fig 1 is an illustration of an exemplary electronic device comprising an acoustic biometric imaging system according to an embodiment of the present invention, in the form of a mobile phone;
Fig 2A is a schematic cross-section view of the acoustic biometric imaging system in fig 1, with the section taken along the line A-A' in fig 1;
Fig 2B is an enlarged view of a portion of the acoustic biometric imaging system in fig 2A;
Fig 3 is a partial cross-section view of the ultrasonic transducer array included in the acoustic biometric imaging system in fig 2A, with the section taken along the line B-B' in fig 1;
Fig 4 is a flow-chart illustrating an example manufacturing method; and
Figs 5A-E schematically illustrate the result of the respective method steps in the flow-chart in fig 4.

### Detailed Description of Example Embodiments

In the present detailed description, various embodiments of the acoustic biometric imaging system according to the present invention are mainly described with reference to an acoustic biometric imaging system comprising a cover glass for a mobile communication device, with an ultrasonic transducer array attached thereto. It should be noted that acoustic biometric imaging systems with many other configurations also fall within the scope defined by the claims. For instance, the transparent device member need not necessarily be a cover glass, and/or the ultrasonic transducer array included in the acoustic biometric imaging system may include fewer or more piezoelectric elements. Moreover, the first and second transducer electrodes may be connectable from the same or different sides of the ultrasonic transducer device.

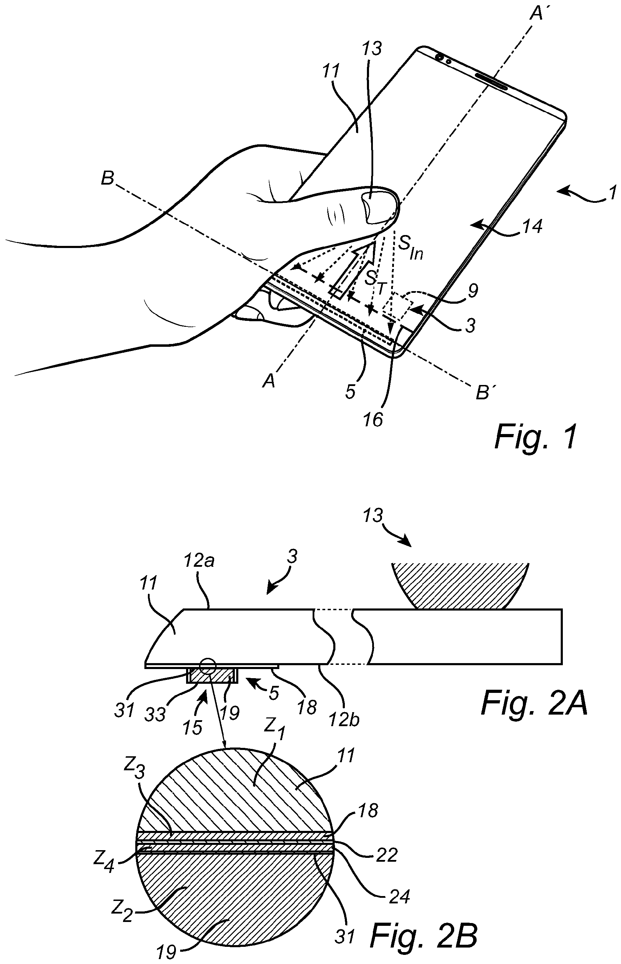

The acoustic biometric imaging system according to embodiments of the present invention may be included in various electronic devices. Fig 1 schematically illustrates a representative electronic device, in the form of a mobile phone 1, comprising an acoustic biometric imaging system 3 according to an embodiment of the present invention.

As is schematically indicated in fig 1, the acoustic biometric imaging system 3 may comprise an ultrasonic transducer array 5, and a controller 9 connected to the ultrasonic transducer array 5.

The ultrasonic transducer array 5 is acoustically coupled to a transparent device member, here cover glass 11, of the electronic device 1 in a first transducer region, corresponding to the extension of the ultrasonic transducer array 5. The user touch, which takes place in a finger touch region 14 laterally spaced apart from the first transducer region 5, is indicated by the thumb 13 in fig 1. An exemplary near zone limit of the finger touch region 14 is schematically indicated by the dashed line 16 in fig 1.

When the acoustic biometric imaging system 3 is in operation, the controller 9 controls one or several piezoelectric element(s) comprised in the ultrasonic transducer array 5 to transmit an acoustic transmit signal S_{T}, indicated by the block arrow in fig 1. Further, the controller 9 controls the ultrasonic transducer array 5 to receive acoustic interaction signals S_{In}, indicated by the dashed arrows in fig 1. The acoustic interaction signals S_{In} are indicative of interactions between the transmit signal S_{T} and the interface between the cover glass 11 and the skin of the user (thumb 13). The acoustic interaction signals S_{In} are transformed to electrical signals by the receiving piezoelectric elements in the ultrasonic transducer array 5, and the electrical signals are processed by the controller 9 to provide a representation of the fingerprint of the user.

The acoustic interaction signals S_{In} are presently believed to mainly be due to so-called contact scattering at the contact area between the cover glass and the skin of the user (thumb 13).

The acoustic transmit signal S_{T} may advantageously be a pulse train of short pulses (impulses), and the acoustic interaction signals S_{In}, which may be measured for different angles by different receiving piezoelectric elements, may then be impulse responses. The impulse response data carried by the acoustic interaction signals S_{In} can be used to reconstruct a representation of the contact area (the fingerprint) using a reconstruction procedure similar to methods used in ultrasound reflection tomography.

It should be understood that the "representation" of the fingerprint of the user may be any information extracted based on the received acoustic interaction signals S_{In}, which is useful for assessing the similarity between fingerprint representations acquired at different times. For instance, the representation may comprise descriptions of fingerprint features (such as so-called minutiae) and information about the positional relationship between the fingerprint features. Alternatively, the representation may be a fingerprint image, or a compressed version of the image. For example, the image may be binarized and/or skeletonized. Moreover, the fingerprint representation may be the above-mentioned impulse response representation.

Fig 2A is a schematic cross-section view of the acoustic biometric imaging system 3 in fig 1, with the section taken along the line A-A' in fig 1, and fig 2B is an enlarged view of a portion of the acoustic biometric imaging system 3 in fig 2A.

Referring first to fig 2A, the transparent device member 11, here cover glass, has a first face 12a to be touched by a finger surface of a user, and a second face 12b opposite the first face 12a. The ultrasonic transducer array 5 comprises a plurality of ultrasonic transducers 15, each comprising a piezoelectric element 19, a first transducer electrode 31, and a second transducer electrode 33. Each of the ultrasonic transducers 15 is acoustically coupled to the second face 12b of the transparent device member 11. As can be seen in fig 2A, the acoustic biometric imaging system 3 further comprises an opaque masking layer 18 arranged between the second face 12b of the transparent device member 11 and the ultrasonic transducers 15 in the ultrasonic transducer array 5. The opaque masking layer 18 render the ultrasonic transducer array 5 invisible from the first face 12a of the transparent device member 11, and can be colored as desired.

Referring now additionally to fig 2B, the exemplary acoustic biometric imaging system 3 in fig 2A further comprises a metal layer 22 deposited on the opaque masking layer 18, and an attachment layer 24 deposited on the metal layer 22. The top electrode 31 of the ultrasonic transducer 15 is bonded to the transparent device member 11 using the attachment layer 24.

As is also indicated in fig 2B, the transparent device member 11 has a first acoustic impedance Z₁, the ultrasonic transducer 15 (the piezoelectric element 19) has a second acoustic impedance Z₂, the opaque masking layer 18 has a third acoustic impedance Z₃, and the attachment layer 24 has a fourth acoustic impedance Z₄.

To provide for a good acoustic coupling between the ultrasonic transducer 15 and the transparent device member 11, the third acoustic impedance Z₃ and the fourth acoustic impedance Z₄ should both have values between the value of the first acoustic impedance Z₁ and the second acoustic impedance Z₂. Since the metal layer 22 and the first transducer electrode 31 can be made very thin (such as less than 1 µm), these layers can be disregarded in view of the acoustic coupling between the ultrasonic transducer 15 and the transparent device member 11.

An ultrasonic transducer 15 comprising a piezoelectric element 19 made of PZT has an acoustic impedance of about 23.6 MRayls for longitudinal waves, and about 14.4 MRayls for shear waves.

Chemically modified glass (such as so-called gorilla glass), which is a suitable material for the transparent device member 11 for use in, for example, a mobile communication device 1, has an acoustic impedance of about 13.7 MRayls for longitudinal waves, and about 8.8 MRayls for shear waves.

For a first exemplary acoustic biometric imaging system using longitudinal waves, the first acoustic impedance Z₁ may thus be around 23.6 MRayls, and the second acoustic impedance Z₂ may be around 13.7 MRayls. This means that the third acoustic impedance Z₃ should be higher than about 13.7 MRayls, and less than about 23.6 MRayls.

For a second exemplary acoustic biometric imaging system using shear waves, the first acoustic impedance Z₁ may be around 14.4 MRayls, and the second acoustic impedance Z₂ may be around 8.8 MRayls. This means that the third acoustic impedance Z₃ should be higher than about 8.8 MRayls, and less than about 14.4 MRayls.

Neither of these ranges can be achieved using conventional polymer ink, which typically has an acoustic impedance of less than 1 MRayls for both longitudinal waves and shear waves.

According to embodiments of the biometric acoustic imaging system 3 of the present invention, the opaque masking layer 18 is instead a vacuum deposited oxide layer formed by a mix of silicon oxide and zirconium oxide.

Silicon oxide (silicon dioxide) has (depending on the density) an acoustic impedance of about 13 MRayls for longitudinal waves, and about 8.5 MRayls for shear waves.

Zirconium oxide (zirconium dioxide) has (depending on the density) an acoustic impedance of about 30 MRayls for longitudinal waves, and about 19 MRayls for shear waves.

By vacuum depositing silicon oxide and zirconium oxide in suitable proportions, it is clear that an opaque layer can be achieved that is within the desired acoustic impedance ranges for longitudinal waves as well as for shear waves. Using Non-Conductive Vacuum Metallization (NCVM), which is, per se, well known in the art for other applications, silicon oxide and zirconium oxide can be provided in suitable proportions. A desired appearance (such as color) of the opaque masking layer 18 can, for example, be achieved by tuning the thickness of the layer.

The attachment layer 24 may suitably, for example, consist of a Sn-Bi alloy, which has an acoustic impedance of 11.3 MRayls for shear waves.

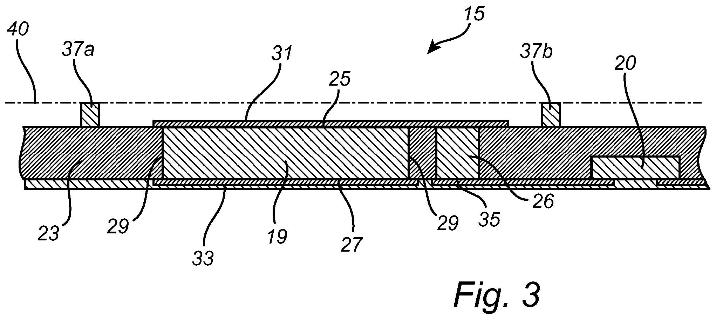

Before turning to an exemplary embodiment of the manufacturing method according to the present invention, an example configuration of the ultrasonic transducers 15 in the biometric acoustic imaging system 3 will be described with reference to fig 3.

As is indicated in fig 3, the piezoelectric element 19 has a first face 25, a second face 27, and side edges 29 extending between the first face 25 and the second face 27. The first transducer electrode 31 can be shaped to directly interconnect the first face 25 of the piezoelectric element 19 with a conductive via 26. As can also be clearly seen in fig 3, the edges 29 of the piezoelectric element 19 are completely covered by the embedding dielectric material 23, and as the embedding dielectric material 23 and the piezoelectric element 19 have been thinned in the same thinning process, the embedding dielectric material 23 is co-planar with the first face 25 of the piezoelectric element 19, at least at the side edges 29 of piezoelectric element. Moreover, the integrated circuit 20, which may, for example be an ultrasound driver circuit for driving at least one piezoelectric element with a relatively high voltage signal, such as 12 V or more, and/or an ultrasound receiver circuit, is completely embedded by the dielectric material 23. As can also be seen in fig 3, the spacers 37a-b define a spacer plane, represented by the line 40 in fig 3, which is spaced apart from the first transducer electrodes 31 and parallel with a plane defined by the first face 25 of the piezoelectric element 19.

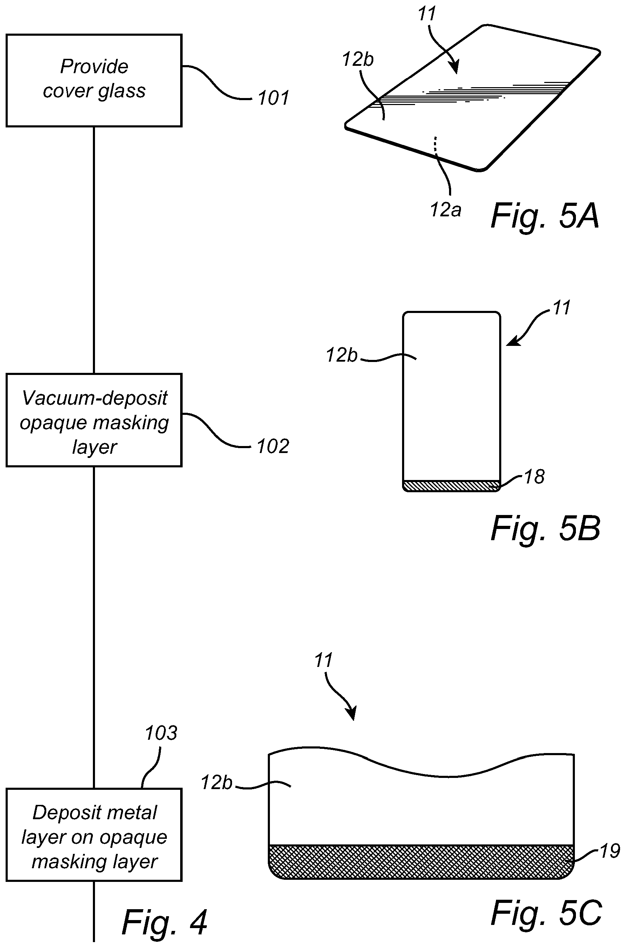

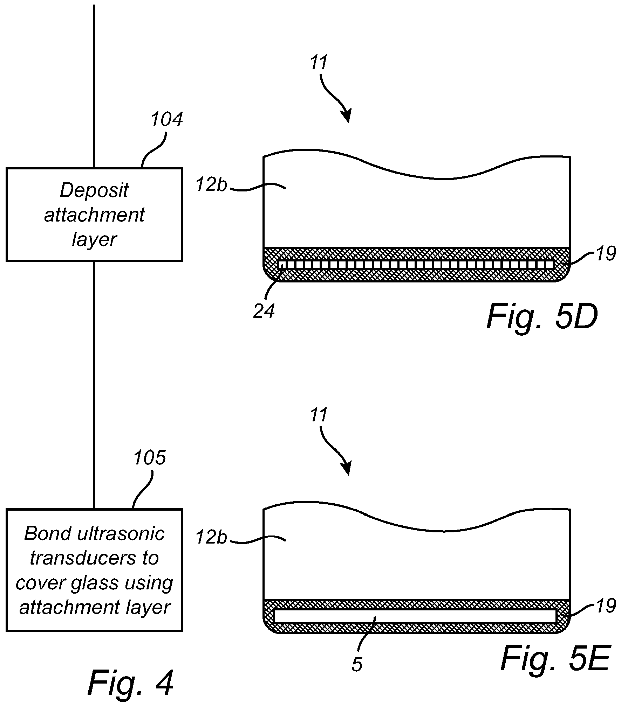

An example method of manufacturing the acoustic biometric imaging system 3 according to embodiments of the invention will now be described with reference to the flow-chart in fig 4, and the accompanying illustrations in figs 5A-E.

In a first step 101, a transparent device member, here cover glass 11 having a first face 12a and a second face 12b is provided.

In the subsequent step 102, an opaque masking layer 18 is vacuum deposited on a portion of the second face 12b of the cover glass 11, using NCVM. As described further above, the proportions of silicon oxide and zirconium oxide, or other suitable oxides or nitrides, are tuned so that the opaque masking layer 18 exhibits an acoustic impedance in a desired range as exemplified further above. As the NCVM process is, per se, well known for other purposes, details of NCVM processing are not provided herein.

In the next step, 103, a thin metal layer 22 is deposited on the opaque masking layer 18, using, for example PVD. The metal layer 22 is provided as an interface layer between the opaque masking layer 18 and the attachment layer 24, that is deposited in step 104.

In the final step 105, an ultrasonic transducer array 5 comprising a plurality of ultrasonic transducers 15 is bonded to the second face 12b of the cover glass 11, using the attachment layer 24.

In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage.

## Claims

1. An acoustic biometric imaging system (3) comprising:
a transparent device member (11) having a first face (12a) to be touched by a finger surface (13) of a user, and a second face (12b) opposite the first face, said transparent device member having a first acoustic impedance (Z₁);
a ultrasonic transducer (15) acoustically coupled to the second face (12b) of said transparent device member (11) in a first transducer region (5) for receiving acoustic signals (S_{In}) conducted by said transparent device member (11) from a finger touch region (14) laterally spaced apart from said transducer region (5), saidfirst ultrasonic transducer (15) having a second acoustic impedance (Z₂);
an opaque masking layer (18) arranged between said transparent device member (11) and said ultrasonic transducer (15) in said transducer region (5), said opaque masking layer having a third acoustic impedance (Z₃) between said first acoustic impedance (Z₁) and said second acoustic impedance (Z₂); and
an attachment layer (24) between said opaque masking layer (18) and said ultrasonic transducer (15), wherein said attachment layer (24) is a Bismuth-based alloy.

2. The acoustic biometric imaging system (3) according to claim 1, wherein said third acoustic impedance (Z₃) is greater than 8 MRayls and less than 24 MRayls.

3. The acoustic biometric imaging system according to claim 1 or 2, wherein said opaque masking layer has a thickness of less than 10 µm.

4. The acoustic biometric imaging system (3) according to any one of the preceding claims, wherein said opaque masking layer (18) is a vacuum deposited layer.

5. The acoustic biometric imaging system (3) according to any one of the preceding claims, wherein said opaque masking layer is (18) an oxide layer.

6. The acoustic biometric imaging system (3) according to claim 5, wherein said opaque masking layer (18) comprises silicon and zirconium.

7. The acoustic biometric imaging system (3) according to any one of the preceding claims, further comprising a metallic layer (22) between said opaque masking layer (18) and said attachment layer (24).

8. The acoustic biometric imaging system (3) according to claim 7, wherein said metallic layer (22) is a vacuum deposited layer.

9. The acoustic biometric imaging system (3) according to any one of the preceding claims, wherein said ultrasonic transducer (15) is a ceramic transducer.

10. The acoustic biometric imaging system (3) according to any one of the preceding claims, wherein said ultrasonic transducer (15) is a shear wave transducer.

11. The acoustic biometric imaging system (3) according to any one of the preceding claims, further comprising:
transducer control circuitry (20) connected to said ultrasonic transducer (15) for receiving, from said ultrasonic transducer, electrical signals indicative of the acoustic signals conducted by said transparent device member (11) from said finger touch region (14).

12. The acoustic biometric imaging system (3) according to claim 11, further comprising processing circuitry (9) connected to said transducer control circuitry (20) and configured to form a representation of said finger surface (13) based on signals from said transducer control circuitry (20).

## Patentansprüche

1. Akustisches biometrisches Bildgebungssystem (3), das umfasst:
ein transparentes Vorrichtungselement (11), das eine erste Fläche (12a) aufweist, die durch eine Fingeroberfläche (13) eines Benutzers berührt werden soll, und eine zweite Fläche (12b) aufweist, die der ersten Fläche gegenüberliegt, wobei das transparente Vorrichtungselement eine erste akustische Impedanz (Z₁) aufweist;
einen Ultraschallwandler (15), der akustisch mit der zweiten Fläche (12b) des transparenten Vorrichtungselements (11) in einer Wandlerregion (5) gekoppelt ist, um akustische Signale (Sᵢₙ) zu empfangen, die durch das transparente Vorrichtungselement (11) von einer Fingerberührungsregion (14) her geleitet werden, die seitlich von der Wandlerregion (5) beabstandet ist, wobei der Ultraschallwandler (15) eine zweite akustische Impedanz (Z₂) aufweist;
eine opake Maskierungsschicht (18), die zwischen dem transparenten Vorrichtungselement (11) und dem Ultraschallwandler (15) in der Wandlerregion (5) angeordnet ist, wobei die opake Maskierungsschicht eine dritte akustische Impedanz (Z₃) zwischen der akustischen Impedanz (Z₁) und der zweiten akustischen Impedanz (Z₂) aufweist; und
eine Anbringungsschicht (24) zwischen der opaken Maskierungsschicht (18) und dem Ultraschallwandler (15), wobei die Anbringungsschicht (24) eine Legierung auf Bismutbasis ist.

2. Akustisches biometrisches Bildgebungssystem (3) nach Anspruch 1,
wobei die dritte akustische Impedanz (Z₃) größer als 8 MRayIs und kleiner als 24 MRayIs ist.

3. Akustisches biometrisches Bildgebungssystem nach Anspruch 1 oder 2, wobei die opake Maskierungsschicht eine Dicke von weniger als 10 um hat.

4. Akustisches biometrisches Bildgebungssystem (3) nach einem der vorangehenden Ansprüche, wobei die opake Maskierungsschicht (18) eine im Vakuum abgeschiedene Schicht ist.

5. Akustisches biometrisches Bildgebungssystem (3) nach einem der vorangehenden Ansprüche, wobei die opake Maskierungsschicht (18) eine Oxidschicht ist.

6. Akustisches biometrisches Bildgebungssystem (3) nach Anspruch 5, wobei die opake Maskierungsschicht (18) Silizium und Zirkonium umfasst.

7. Akustisches biometrisches Bildgebungssystem (3) nach einem der vorangehenden Ansprüche, das des Weiteren eine Metallschicht (22) zwischen der opaken Maskierungsschicht (18) und der Anbringungsschicht (24) umfasst.

8. Akustisches biometrisches Bildgebungssystem (3) nach Anspruch 7, wobei die Metallschicht (22) eine im Vakuum abgeschiedene Schicht ist.

9. Akustisches biometrisches Bildgebungssystem (3) nach einem der vorangehenden Ansprüche, wobei der Ultraschallwandler (15) ein keramischer Wandler ist.

10. Akustisches biometrisches Bildgebungssystem (3) nach einem der vorangehenden Ansprüche, wobei der Ultraschallwandler (15) ein Scherwellenwandler ist.

11. Akustisches biometrisches Bildgebungssystem (3) nach einem der vorangehenden Ansprüche, das des Weiteren umfasst:
eine Wandlersteuerungsschaltung (20), die mit dem Ultraschallwandler (15) verbunden ist, um von dem Ultraschallwandler elektrische Signale zu empfangen, die die akustischen Signale angeben, die durch das transparente Vorrichtungselement (11) von der Fingerberührungsregion (14) her geleitet werden.

12. Akustisches biometrisches Bildgebungssystem (3) nach Anspruch 11, das des Weiteren eine Verarbeitungsschaltung (9) umfasst, die mit der Wandlersteuerungsschaltung (20) verbunden und so konfiguriert ist, dass sie eine Darstellung der Fingeroberfläche (13) auf der Grundlage von Signalen von der Wandlersteuerungsschaltung (20) bildet.

## Revendications

1. Système d'imagerie biométrique acoustique (3) comprenant :
un élément de dispositif transparent (11) comportant une première face (12a) à toucher par une surface de doigt (13) d'un utilisateur, et une deuxième face (12b) à l'opposé de la première face, ledit élément de dispositif transparent présentant une première impédance acoustique (Z₁) ;
un transducteur ultrasonique (15) couplé acoustiquement à la deuxième face (12b) dudit élément de dispositif transparent (11) dans une région de transducteur (5) pour recevoir des signaux acoustiques (Sᵢₙ) conduits par ledit élément de dispositif transparent (11) depuis une région de toucher de doigt (14) latéralement espacée de ladite région de transducteur (5), ledit transducteur ultrasonique (15) présentant une deuxième impédance acoustique (Z₂) ;
une couche de masquage opaque (18) agencée entre ledit élément de dispositif transparent (11) et ledit transducteur ultrasonique (15) dans ladite région de transducteur (5), ladite couche de masquage opaque présentant une troisième impédance acoustique (Z₃) entre ladite première impédance acoustique (Z₁) et ladite deuxième impédance acoustique (Z₂) ; et
une couche de fixation (24) entre ladite couche de masquage optique (18) et ledit transducteur ultrasonique (15), dans lequel ladite couche de fixation (24) est un alliage à base de bismuth.

2. Système d'imagerie biométrique acoustique (3) selon la revendication 1, dans lequel ladite troisième impédance acoustique (Z₃) est plus grande que 8 MRayls et plus petite que 24 MRayls.

3. Système d'imagerie biométrique acoustique selon la revendication 1 ou 2, dans lequel ladite couche de masquage opaque présente une épaisseur de moins de 10 µm.

4. Système d'imagerie biométrique acoustique (3) selon l'une quelconque des revendications précédentes, dans lequel ladite couche de masquage opaque (18) est une couche déposée sous vide.

5. Système d'imagerie biométrique acoustique (3) selon l'une quelconque des revendications précédentes, dans lequel ladite couche de masquage opaque (18) est une couche d'oxyde.

6. Système d'imagerie biométrique acoustique (3) selon la revendication 5, dans lequel ladite couche de masquage opaque (18) comprend du silicium et du zirconium.

7. Système d'imagerie biométrique acoustique (3) selon l'une quelconque des revendications précédentes, comprenant en outre une couche métallique (22) entre ladite couche de masquage opaque (18) et ladite couche de fixation (24).

8. Système d'imagerie biométrique acoustique (3) selon la revendication 7, dans lequel ladite couche métallique (22) est une couche déposée sous vide.

9. Système d'imagerie biométrique acoustique (3) selon l'une quelconque des revendications précédentes, dans lequel ledit transducteur ultrasonique (15) est un transducteur en céramique.

10. Système d'imagerie biométrique acoustique (3) selon l'une quelconque des revendications précédentes, dans lequel ledit transducteur ultrasonique (15) est un transducteur à ondes de cisaillement.

11. Système d'imagerie biométrique acoustique (3) selon l'une quelconque des revendications précédentes, comprenant en outre
une circuiterie de commande de transducteur (20) reliée audit transducteur ultrasonique (15) pour recevoir, depuis ledit transducteur ultrasonique, des signaux électriques indicatifs des signaux acoustiques conduits par ledit élément de dispositif transparent (11) depuis ladite région de toucher de doigt (14).

12. Système d'imagerie biométrique acoustique (3) selon la revendication 11, comprenant en outre une circuiterie de traitement (9) reliée à ladite circuiterie de commande de transducteur (20) et configurée pour former une représentation de ladite surface de doigt (13) sur la base de signaux provenant de ladite circuiterie de commande de transducteur (20).
